# EUROPEAN PATENT SPECIFICATION

(11) **EP 4 454 588 B1**
(45) Date of publication and mention of the grant of the patent: **11.02.2026**
(21) Application number: 23169542.0
(22) Date of filing: 24.04.2023
(51) Int. Cl.: A61B 34/20, A61B 34/10, A61B 90/50, A61B 17/00, A61B 90/00, G09B 23/28

(54) **SYSTEM AND METHODS FOR EXTENDED REALITY VISUALIZATION OF SURGICAL PROCEDURAL STEPS FOR TRAINING AND TESTING OF X-RAY IMAGE BASED SURGICAL NAVIGATION SYSTEM**
SYSTEM UND VERFAHREN ZUR ERWEITERTEN REALITÄTSVISUALISIERUNG CHIRURGISCHER PROZEDURALER SCHRITTE ZUM TRAINIEREN UND TESTEN EINES RÖNTGENBILDBASIERTEN CHIRURGISCHEN NAVIGATIONSSYSTEMS
SYSTÈME ET PROCÉDÉS DE VISUALISATION EN RÉALITÉ ÉTENDUE D'ÉTAPES CHIRURGICALES PROCÉDURALES POUR L'APPRENTISSAGE ET LE TEST D'UN SYSTÈME DE NAVIGATION CHIRURGICALE BASÉ SUR UNE IMAGE À RAYONS X

(43) Date of publication of application: 30.10.2024
(73) Proprietor: metamorphosis GmbH, 33184 Altenbeken (DE)
(72) Inventor: Blau, Arno, 72336 Balingen (DE); Schlenker, Julian, 33098 Paderborn (DE)
(74) Representative: LKGLOBAL Partnerschaftsgesellschaft mbB

(56) References cited:
- EP-A1- 4 092 624
- US-A1- 2020 275 988
- US-A1- 2021 369 230
- US-A1- 2022 058 797

## Description

### FIELD OF INVENTION

The invention relates to the fields of extended reality visualization. Further, the invention relates to computer-assisted surgery as well as to robotic-assisted surgery. The systems and methods described herein can be applied to assist in performing steps in surgical procedures, both in a training setup and in a real surgical procedure. The methods can be implemented as computer program product which can be executed on a processing unit of a system.

### BACKGROUND OF THE INVENTION

Surgical navigation or Computer assisted surgery (CAS) has become a commonly performed procedure in order to improve patient outcome and to reduce X-ray exposure to patient and operation room staff. However, there may be usability issues resulting in misunderstanding. For example, the surgeon, the nurse, or the x-ray technician do not understand what the CAS system currently expects as a next step. Even when the user understands what the system expects, it may still be a problem to know how to follow the instructions correctly. As one example, C-arm positioning in order to acquire the desired X-ray image may be a hassle resulting in more exposure than needed and more procedural time. A CAS system may know how the C-arm should be repositioned in order the acquire a next X-ray image but displaying that information may cause misunderstanding.

US 2020/0275988 A1 is directed to a system and method for image to world registration for medical reality applications, using a world spatial map. Any point in a fluoroscopic image is linked to its corresponding position in the visual world using spatial mapping with a head mounted display (HMD) (world tracking).

### SUMMARY OF THE INVENTION

When using a CAS system, it may be of interest to test as to how the CAS system works and as to whether information provided by the system can be validated. The question may be whether the CAS system work correctly.

At least the one or the other of the mentioned problems are mitigated or solved by the subject matter according to each of the independent claims. Further embodiments are described in the respective dependent claims.

In general, a system in accordance with the invention comprises an extended reality device as well as a processing unit for executing a computer program product. By means of the extended reality device, objects in a room can be tracked and based on processing of tracking data related to those objects and based on processing of simulated X-ray imaging data, additional information can be visualized in the field of view of the user.

It will be understood that extended reality devices cover all devices on the extended reality spectrum and are therefore a superset of virtual reality, augmented reality und mixed reality devices. This represents all devices which can be worn by a user and can (1) alter the perception of the user via any sense - including vision, hearing, olfaction, gustation and tactile perception - and (2) can track its environment via a variety of sensor information - including cameras, depth sensors, accelerometers and magnetometers. Those sensors may track the environment including an imaging device and/or at least one object like an anatomy model, a tool, or an implant. It is noted that the tracking information allows for a differentiation of objects and/or devices. For example, the extended reality device may comprise a pair of glasses through which the user may see the environment, but which are configured to visualize information or a virtual representation of an object in the field of view of the user.

A method for extended reality training with or testing of an X-ray imaging-based computer assisted surgical device, when performed by a processing unit of a system, may generally comprise the steps of determining a 3D pose of an X-ray imaging device in a coordinate system, determining a 3D pose of an object in the coordinate system, generating a simulated X-ray projection image of the object, wherein the simulated X-ray projection image is generated on the basis of the determined 3D pose of the X-ray imaging device and on the basis of the determined 3D pose of the object, interpreting the simulated X-ray projection image, and providing information to a user by an extended reality device, wherein the extended reality device is configured to track its 3D pose in the coordinate system, wherein the provided information is based on the interpretation of the simulated X-ray projection image.

According to an embodiment, a system comprising an extended reality device and a processing unit connected to the same, may be caused to perform a method including the steps of determining a 3D pose of an X-ray imaging device, wherein the 3D pose of the X-ray imaging device may be based on the tracking information received from the extended reality device, when the X-ray imaging device is a real X-ray imaging device, and of determining a 3D pose of an object, wherein the 3D pose of the object may be based on the tracking information received from the extended reality device, when the object is a real object. It will be understood that both the X-ray imaging device and the object may be virtual so that the 3D pose may respectively be determined by a processing unit of the system without external tracking information. In the context of the present disclosure, "3D pose" means a 3D position and orientation determined in relation to a coordinate system.

Further, the step of generating a simulated X-ray projection image of the object may be performed, wherein the simulated X-ray projection image is generated on the basis of the determined 3D pose of the X-ray imaging device and on the basis of the determined 3D pose of the object. There may be more than one object visible in the X-ray image and each of these objects may or may not be tracked by the extended reality device.

The method may further comprise the steps of interpreting the simulated X-ray projection image and of providing information to the user, wherein the information is based on the interpretation of the simulated X-ray projection image.

Part of the interpretation may be a determination of relative spatial position between objects or between objects and X-ray image device. This spatial relation may be compared with the expected spatial relation based on the 3D pose of objects and/or the 3D pose of the X-ray imaging device. Thus it can also be used for validation or testing purposes, e.g. validating the algorithms for interpretation of the X-ray images based on the assumption that the determination of the 3D pose(s) performed by the extended reality device is correct or validating the determination of poses of object(s) and/or X-ray imaging device performed by the extended reality device based on the assumption that the interpretation of the simulated X-ray image is correct. Also, a combination of both may be possible.

A single X-ray image may be required to validate correctness of real time tracking information, i.e., to determine whether or not the last registration (i.e., determination of the 3D pose) of the object relative to another object is still valid. In other words, the proposed systems and methods (implemented as computer program product) may be configured to determine the validity of 3D spatial relation based on a single X-ray image depicting at least part of an object.

That is, the determination of a 3D pose of an object results in a simulated X-ray projection image, and based on an interpretation of that projection image, information about the spatial arrangement of the object can be provided. For training purposes, such information may be used to guide a user through a procedure. For testing of the system, the information may be compared with the determined 3D pose. Based on such comparison, an indication can be provided whether the information provided based on the interpretation of a projection image are sufficiently correct, i.e., are within a predetermined deviation range of an expected value.

The interpretation of the simulated X-ray image may comprise at least one of (i) determining a geometrical aspect of the object, (ii) applying a model of the object, (iii) identifying a point of interest, (v) measuring dimensions, (vi) measuring distances, (vii) taking into account imaging characteristics, (viii) taking into account object characteristics, and (ix) determining relative spatial positioning information between at least one imaged object and/or the X-ray imaging device.

Taking into account imaging characteristics may include applying a priori knowledge about physical effects of distortion of X-ray images caused by earth magnetic field or pillow distortion. Taking into account object characteristics may include a priori knowledge about a typical mechanical deformation of a tool e.g. like bending of a drill bit or mechanical play e.g. between a drill bit and a drill sleeve.

Determining of a relative spatial positioning information between two or more objects is disclosed in more detail in patent application EP 19 217 245 and patent application EP 21 175 405, e.g., in the claims of these patent applications. Furthermore, imaging characteristics as well as object characteristics which may be relevant when processing X-ray images, are described in these documents in more detail. These detailed disclosures are incorporated herein by reference.

The determined spatial relation between the at least one imaged object and/or the X-ray imaging device may be compared with the 3D pose of the X-ray imaging device and/or with the 3D pose of the object as generally determined according to an embodiment of the disclosure.

Furthermore, the determination of the 3D pose of the X-ray imaging device and/or the determination of the 3D pose of the object may be adjusted based on the interpretation of the simulated X-ray projection image. Typically, the information provided to the user may be adjusted accordingly.

The method may further comprise the step of determining a representation of the X-ray imaging device and/or of the object, wherein the representation of the X-ray imaging device and/or of the object is based on the tracked 3D pose of the extended reality device and on the determined 3D pose of the X-ray imaging device and/or on the determined 3D pose of the object. Further, the representation may be visualized by the extended reality device.

In the context of this disclosure, a representation is to be understood as, e.g., a visualization of at least an aspect of the X-ray imaging device or the object. An aspect may be a point or an axis, but also be an outline or even a rendered surface. In other words, a representation of a virtual device or object may provide enough information to a user which allow for a recognition of the device or object itself as well as a 3D position and 3D orientation thereof.

Several different combinations of objects and X-ray devices are encompassed. The object may be at least one object out of the group consisting of (i) at least a part of a virtual anatomy, (ii) at least a part of a real anatomy model, (iii) at least a part of a virtual tool, (iv) at least a part of a real tool, (v) at least a part of a real implant, and (vi) at least a part of a virtual implant. The X-ray imaging device may also be a real X-ray imaging device or a virtual X-ray imaging device. It is noted that the mentioned real anatomy model may be an artificially constructed part of an anatomy dummy or mock-up.

It will be understood that the method may comprise the step of tracking the at least one object and/or the X-ray imaging device by the extended reality device, when the at least one object is a real object and/or the X-ray imaging device is a real X-ray imaging device.

The information provided to the user may comprise at least one information out of the group consisting of (i) an advise how to reposition the X-ray imaging device, (ii) a visualization of a position of a virtual image plane of the X-ray image, (iii) a visualization of a position of a virtual image plane of a previous simulated X-ray image, (iv) an advise for a next step of the procedure.

The information which is based on the interpretation of the simulated X-ray image may be provided to the user by means of the extended reality device.

With respect to the provision of information to the user, it is noted that the information which is based on the interpretation of the simulated X-ray image may be sensed by vision, hearing, olfaction, gustation and/or tactile perception. For example, the system may provide an instruction accustically or may provide an alert optically by a change of a color of light. It will be understood that a virtual X-ray imaging device and/or a virtual object may be visualized on the extended reality device, whereas some information like measurement values may also be provided on a stationary monitor.

The method may further comprise the steps of receiving an input and updating the information provided to the user based on the input.

In the context of this disclosure, an input may be understood as any kind of active interaction with the system. For example, a user may change the 3D pose of a device and/or object and the extended reality device tracks that change, wherein the new tracking information may be considered as input to the system. On the one hand, a user may change the 3D pose of a real device and/or real object which can be tracked by the extended reality device. On the other hand, a user may change the 3D pose of a virtual device and/or virtual object by means of a tracked hand gesture, a controller or any other suitable computer input device.

As described above, the method may be performed in a complete virtual space, with the X-ray imaging device as well as any object, i.e., tool, implant, anatomy, being virtually generated and visualized on a screen of an extended reality device. Such virtual devices and objects may be moved due to an input or as a result of the interpretation of a simulated X-ray image. On the other hand, any of the imaging device and the object(s) may be real and may be tracked by the extended reality device. Such real devices and objects may be visible through glasses of the extended reality device and may, thus, not be shown on the screen in form of a virtual representation.

However, a combination is also possible. A representation of a real X-ray imaging device and/or of a real object may be visualized by the extended reality device, so that the representation is visible as an overlay on the X-ray imaging device and/or on the object being visible at the same time through the glasses of the extended reality device.

Such an overlay may provide the possibility to recognize whether the tracking and/or determining of the 3D pose of the device or object is sufficiently correct. A deviation of the virtual representation and the real object may easily be recognized.

As mentioned previously, the processing unit of the system may be caused to update a tracked 3D pose of a device and/or object. Such an update may be visualized by a change of the representation of the device and/or object. Thus, the representation may be manipulated due to an input of the user. On the other hand, the system may manipulate the representation autonomously based on the interpretation of the simulated X-ray image. By way of autonomously manipulating the representation, the system may provide suggestions to the user as to how to further proceed. E.g. the system computes and displays an virtual overlay of an desired (other) position of an X-ray imaging device on the current position of the X-ray imaging device based on the interpretation of the simulated X-ray image.

Besides using the system for training of a user, it is also possible to use the input of the user to generate training data for further enhancement of the system. For example, the system may automatically adopt to preferences of the user.

It is noted that the processing unit of the system as described herein may be realized by only one processor performing all the steps of the process, or by a group or a plurality of processors, which need not be located at the same place. For example, cloud computing allows a processor to be placed anywhere. For example, a processing unit may be divided into a first sub-processor that controls interactions with the user, including an extended reality device for visualizing results, and a second sub-processor (possibly located elsewhere) that performs all computations. The first sub-processor or another sub-processor may also control movements of, for example, an X-ray imaging device. A processing unit integrated in the extended reality device may control any tracking of objects as well as any visualizations on the screen of the extended reality device.

The system may further comprise storage means providing a database for storing, for example, X-ray images. It will be understood that such storage means may also be provided in a network to which the system may be connected, and that data related to a neural net may be received over that network. Furthermore, the device may comprise an imaging unit for generating at least one 2D X-ray image, wherein the imaging unit may be capable of generating images from different directions.

It is noted that all references to C-arm (imaging device) movements in this disclosure always refer to a relative repositioning between C-arm and model of the patient. Hence, any C-arm translation or rotation may in general be replaced by a corresponding translation or rotation of the model of the patient/OR table, or a combination of C-arm translation/rotation and model of the patient/table translation/rotation. Information about changes in positioning may be provided by the tracking system of the extended reality device. Alternatively or additionally, such information may be provided by positioning sensory devices in a robotic steered model of the patient table and/or robotic steered imaging device, or it may be provided by an optical navigation system that tracks reference frames that are attached to the patient table or an anatomy model, and/or the imaging device. The system may compute C-arm adjustments and/or model of the patient adjustments. It is furthermore noted that all references to a C-arm may analogously apply to a G-arm, O-arm, or similar.

The methods and techniques disclosed herein may be used in a system that supports a human user or surgeon, or they may also be used in a system where some or all of the steps are performed by a robot. Hence, references to a "user" or "surgeon" in this patent disclosure may refer to a human user as well as a robotic surgeon, a mechanical support device, or a similar apparatus. Similarly, whenever it is mentioned that instructions are given how to adjust the C-arm, it is understood that such adjustments may also be performed without human intervention, i.e., automatically, by a robotic C-arm, by a robotic table, or they may be performed by operation room staff with some automatic support. It is noted that because a robotic surgeon and/or a robotic C-arm may operate with higher accuracy than humans, iterative procedures may require fewer iterations, and more complicated instructions (e.g., combining multiple iteration steps) may be executed. A key difference between a robotic and a human surgeon is the fact that a robot may keep a tool perfectly still in between acquisition of two X-ray images.

Like automatically controlled movements of real objects and/or real devices, it is possible to provide a virtual representation of the object and/or device in the field of view of the user by means of the extended reality device. The object and/or device may, thus, be virtually moved, wherein the movement is controlled by the computer program product executed by the processing unit.

The computer program product may preferably be loaded into the main memory of a data processor during execution. The data processor or processing unit of a system according to an embodiment may thus be equipped to carry out at least a part of the described process. Further, the disclosure may relate to a computer-readable medium such as a CD-ROM on which the disclosed computer program product may be stored. However, the computer program product may also be presented over a network like the World Wide Web and can be downloaded into the main memory of the data processor from such a network for instant execution or can be downloaded into non-volatile memory of the data processor for execution at a later point. Furthermore, the computer program product may also be executed on a cloud-based processor, with results presented over the network.

It is noted that prior information about an object (e.g., the size and type of a drill or a chisel) may be obtained by simply scanning of a packaging (e.g., the barcode) or any writing on the object itself, before or during the performance of method steps.

As should be clear from the above description, an aspect may be a processing of X-ray image data, allowing an automatic interpretation of visible objects or determining important regions in an X-ray image. The methods described herein are to be understood as methods for training of a user in applying the system or for testing the system. Consequently, the method may not include any step of treatment of an animal or human body by surgery.

It is further noted that embodiments are described with reference to different subject-matters. In particular, some embodiments are described with reference to method-type claims (computer program product) whereas other embodiments are described with reference to apparatus-type claims (system/device). However, a person skilled in the art will gather from the above and the following description that, unless otherwise specified, any combination of features belonging to one type of subject-matter as well as any combination between features relating to different subject-matters is considered to be disclosed with this application.

It is further noted, that the above-described system could be used as a game just for fun or for a competition.

### BRIEF DESCRIPTION OF THE DRAWINGS

The aspects defined above and further aspects, features and advantages of the present invention can also be derived from the examples of the embodiments to be described hereinafter and are explained with reference to examples of embodiments also shown in the figures.
Fig. 1 illustrates a situation in which a user utilizes a system as disclosed herein.
Fig. 2 is a flowchart of a method as disclosed herein.

### DETAILED DESCRIPTION OF THE EMBODIMENTS

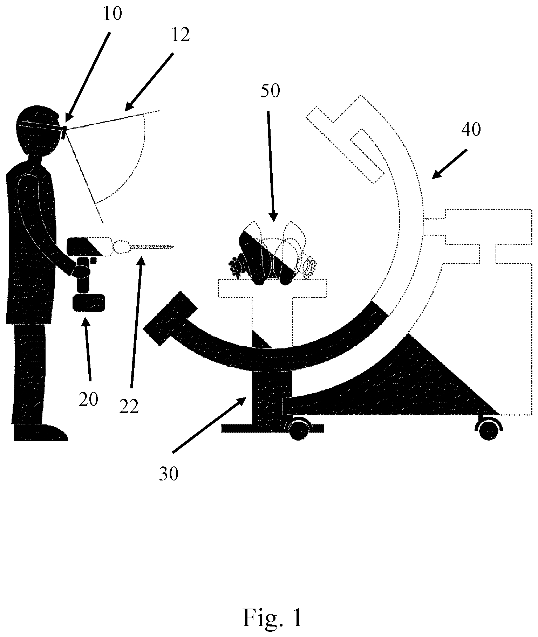

Figure 1 illustrates a situation in which the system and method as disclosed herein can be utilized. An extended reality device 10 may be a head mounted device which comprises sensors for tracking objects in the room. Such objects may be a tool like a drilling machine 20 with a drill bit 22, an operation table 30, a C-arm based X-ray imaging device 40 and/or a model of a patient 50 or part of an anatomy model. It will be understood that it is not necessary that the whole of each object is tracked by the sensors of the extended reality device. For example, it may be sufficient to detect the radiation source and/or the radiation detector of the imaging device to determine a 3D pose of the imaging device, i.e., a 3D position and 3D orientation of the imaging device. It may otherwise be sufficient to detect the radiation detector together with at least a part of the C-arm and the base of the imaging device to allow for a determination of the 3D pose of the device. Comparably, only a part of the table 30 or of the tool 20 must be detected by the tracking sensors.

One way of interpreting figure 1 is that the dark portions, i.e., the portions illustrated as black surfaces, of the objects and of the devices are outside of the tracking, and the light portions, i.e., the portions illustrated by dotted outlines, of the objects and of the devices are those portions which are detected by the sensors of the extended reality device, so that the objects and devices can be tracked and a 3D pose thereof can be determined.

Knowing the 3D pose of the imaging device 40 as well as the 3D pose of the model of the patient 50 allows for the generation of a simulated X-ray image. On the one hand, such a simulated X-ray image may be used to provide an impression whether a real X-ray image would visualize an intended portion of a patient from an intended imaging direction. This may be assessed automatically by the system. On the other hand, the simulated X-ray image allows for training with the system. Based on such a simulated X-ray image, the system may provide information regarding the next step of treatment procedure.

In an alternative situation, at least one of the objects and devices in figure 1 is not present in a room but is shown as a virtual representation on the display of the extended reality device 10. In such a situation, a user may likewise position the imaging device relative to an anatomy or anatomy model and may be provided with a simulated X-ray image allowing an assessment of the treatment procedure. For example, the patient 50 is not really on the table 30 or, instead of a patient, a real training model of the patient may be on the table, wherein the user is provided with a virtual visualization of a patient, possibly as an overlay on the real training model. Thus, the user may position the tool 20 only as if there is a patient. Nevertheless, the system is configured to provide a simulated X-ray image on the basis of which a training of the procedure is possible, utilizing the system.

According to another example, the model of the patient and the model of the drilling machine can be visualized by the augmented reality while e.g. the drilling machine is tracked by the augmented reality device, so that a movement of the real drilling machine will result in an updated pose of the virtual model of the drilling machine in real time. On the other hand, the model of the patient may not be tracked by the augmented reality device. The information derived by interpretation of the simulated X-ray image may be used to determine the pose of the model of the patient relative to the pose of the X-ray imaging device at point in time 1 and the information of the pose of the model of the patient may be updated based on the assumption that the position and orientation of the model of the patient does not change relative to the coordinate system until a next X-ray image is simulated at point in time 3. In this scenario, for the simulation of this next X-ray image, the pose of the model of the patient may be changed in order to simulate an unintended, not tracked movement of a patient. Thus, the navigation information which might be inaccurate at point in time 2 is updated based on interpretation of the simulated X-ray image at point in time 3 and, thus, is accurate based on further real time tracking based information of the pose of the drilling machine provided by the extended reality device after point in time 3 unless there is a new unintended movement of the not tracked model of the patient.

As a further example, the table 30, the imaging device 40 as well as the patient 50 may be provided as virtual representations on the extended reality device 10. In such a situation, the user may virtually interact with, e.g., the virtual imaging device so as to position the same relative to the virtual patient on the virtual table. The system, then, provides a simulated X-ray image based on the 3D pose of the imaging device and the 3D pose of the virtual patient, and the system assesses the image so as to provide information, e.g., about a position and orientation of the tool or about a path like a drilling path.

Another way of interpreting figure 1 is that the objects or devices either are visualized as virtual representation by dotted lines showing the outlines or are visible as real objects or devices shown in figure 1 as solid black portions. It may be understood that also a virtual representation of an object may be visualized on the extended reality device as an overlay on the real object in the field of view 12 of the user.

As described by way of examples, the system in accordance with the present disclosure allows for a complete virtual training situation of a treatment of a patient, as well as any hybrid set up with objects being real and others being virtual. In any case, the system is configured to provide a simulated X-ray image as well as an assessment of the content of the image.

In consequence, the system may be utilized to virtually perform a treatment of a patient, before the patient is actually treated, i.e., may be utilized when planning a treatment. The system may also be utilized after a real treatment of a patient using the data for learning and teaching purposes. In that case there may at least one real X-ray images of the previous surgical procedure are interpreted by the system and the user is asked to follow the system instructions in order to observe if and how the next required step in surgery (e.g. repositioning of an object or X-ray device) could have been performed better (e.g. faster) by the human user/surgical robot.

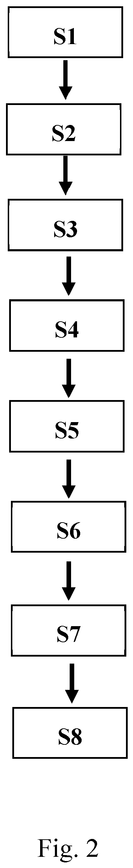

In the following, a method is described with reference to figure 2 showing a flowchart.

It will be understood that steps of methods described herein, and in particular of methods described in connection with a workflow, e.g., as visualized in the figure, are major steps, wherein these major steps might be differentiated or divided into several sub-steps. Furthermore, additional sub-steps might be between these major steps. It will also be understood that only part of the whole method may constitute the invention, i.e. steps may be omitted or summarized.

When utilizing an extended reality device, an X-ray imaging-based computer assisted surgical procedure may comprise the following steps.

In step S1, a 3D pose of an X-ray imaging device is determined. The 3D pose of the X-ray imaging device may be based on tracking information received from the extended reality device. It will be understood that the 3D pose of the imaging device is generated by the system based on a computer simulation in case the imaging device is actually not present.

In step S2, a 3D pose of an object is determined. The 3D pose of the object may be based on tracking information received from the extended reality device. Also here, the 3D pose may be computer generated for a virtual object.

In step S3, a simulated X-ray image of the object is generated, wherein the simulated X-ray image is generated on the basis of both the determined 3D pose of the X-ray imaging device and the determined 3D pose of the object.

In step S4, the simulated X-ray image is interpreted by the system. The computer program product executed by the processing unit of the system may assess the information in the simulated X-ray image. The object may be classified and localized in relation to the imaging device on the basis of the simulated X-ray image. In case of a mock-up bone, fractures may be identified.

In step S5, information to a user is provided, wherein the information is based on the interpretation in S4. For example, suggestions may be provided to the user as to how to treat fractures. When using a tool, a spatial relation of the tool relative to the mock-up bone may be determined and, e.g., a desired insertion path for the tool may be provided and the values (like angles in degree or offsets in mm) of a potential deviation based on current position of the tool from this insertion path may be provided.

In step S6, which is an optional step, a representation of the X-ray imaging device and/or of the object is determined. The representation of the X-ray imaging device and/or of the object may be based on the point of view of the extended reality device and on the determined 3D pose of the X-ray imaging device and/or on the determined 3D pose of the object. As mentioned above, the representation may be visualized on the extended reality device either alone in case the imaging device and/or the object is not present in the field of view of the user, or as an overlay in case the imaging device and/or the object is present. It may be noted that in case the imaging device and/or the object is present such an overlay may provide the advantage that the user immediately recognizes whether the tracking of the imaging device and/or of the object is accurate.

In step S7, an input is received. The information may be updated which is provided to the user, based on the input. An input means may be, e.g., a computer keyboard, a computer mouse or a touch screen, to control a pointing device like a cursor on a monitor screen, which may be included in the device. The device may also comprise a camera or a scanner to read the labeling of a packaging or otherwise identify an implant or surgical tool. A camera may also enable the user to communicate with the device visually by gestures or mimics, e.g., by virtually touching (including drag-and-drop) devices displayed, e.g., on the extended reality device. The device may also comprise a microphone and/or loudspeaker and communicate with the user acoustically.

In step S8, the representation of the X-ray imaging device and/or of the object may be manipulated. On the one hand, the system may automatically adapt the visualization of the representation depending on the movement of the user and, thus, of the extended reality device. On the other hand, the representation may be manipulated based on an input of the user. For example, the user may virtually move the imaging device and/or the object. While embodiments have been illustrated and described in detail in the drawings and aforegoing description, such illustrations and descriptions are to be considered illustrative or exemplary and not restrictive.

Other variations to the disclosed embodiments can be understood and effected by those skilled in the art in practising the claimed invention, from a study of the drawings, the disclosure and the appended claims. **In** the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures can not be used to advantage.

## Claims

1. A method for extended reality training with or testing of an X-ray imaging-based computer assisted surgical device, the method comprising the steps of:
determining a 3D pose of an X-ray imaging device in a coordinate system,
determining a 3D pose of an object in the coordinate system, and
generating a simulated X-ray projection image of the object, wherein the simulated X-ray projection image is generated on the basis of the determined 3D pose of the X-ray imaging device and on the basis of the determined 3D pose of the object,
interpreting the simulated X-ray projection image, and
providing information to a user by an extended reality device, wherein the extended reality device is configured to track its 3D pose in the coordinate system, wherein the provided information is based on the interpretation of the simulated X-ray projection image.

2. The method of claim 1, wherein the step of interpreting of the simulated X-ray projection image comprises at least one out of the group consisting of (i) determining a geometrical aspect of the object, (ii) applying a model of the object, (iii) identifying a point of interest, (v) measuring dimensions, (vi) measuring distances, (vii) taking into account object characteristics, (viii) taking into account imaging characteristics.

3. The method of any one of claims 1 and 2, wherein the step of interpreting of the simulated X-ray projection image comprises determining a spatial relation between at least one imaged object and/or the X-ray imaging device.

4. The method of claim 3, further comprising the step of comparing the spatial relation between the at least one imaged object and/or the X-ray imaging device with the determined 3D pose of the X-ray imaging device and/or with the determined 3D pose of the object.

5. The method of any one of claims 1 to 4, further comprising the step of adjusting the determination of the 3D pose of the X-ray imaging device and/or the determination of the 3D pose of the object based on the interpretation of the simulated X-ray projection image, and wherein the information provided to the user is adjusted based on the interpretation of the simulated X-ray image.

6. The method of any one of claims 1 to 5, further comprising the step of determining a representation of the X-ray imaging device and/or of the object, wherein the representation is based on the tracked 3D pose of the extended reality device and on the determined 3D pose of the X-ray imaging device and/or on the determined 3D pose of the object, and visualizing the representation by the extended reality device.

7. The methods of any one of claims 1 to 6, wherein the object is at least one object out of the group consisting of (i) at least a part of a virtual anatomy, (ii) at least a part of a real anatomy model, (iii) at least a part of a virtual tool, (iv) at least a part of a real tool, (v) at least a part of a real implant, (vi) at least a part of a virtual implant, and wherein the X-ray imaging device is a real X-ray imaging device or a virtual X-ray imaging device.

8. The method of claim 7, further comprising the step of tracking the at least one object and/or the X-ray imaging device by the extended reality device, when the at least one object is a real object and/or the X-ray imaging device is a real X-ray imaging device.

9. The method of any one of claims 1 to 8, wherein the information provided to the user comprises at least one information out of the group consisting of (i) an advise how to reposition the X-ray imaging device, (ii) a visualization of a position of a virtual image plane of the X-ray image, (iii) a visualization of a position of a virtual image plane of a previous simulated X-ray image, (iv) an advise for a next step of the procedure.

10. The method of any one of claims 1 to 9, further comprising the steps of receiving an input and updating the information provided to the user based on the input.

11. The method of any one of claims 1 to 10, wherein the representation of the X-ray imaging device and/or of the object is visualized by the extended reality device, so that the representation is visible as an overlay on the X-ray imaging device and/or the object, when the object is a real object and/or the X-ray imaging device is a real X-ray imaging device visible through the extended reality device.

12. A computer program product including sets of instructions causing a processing unit to perform the steps of the method according to any one of claims 1 to 11, when the computer program product is executed on the processing unit.

13. A system for extended reality training with or testing of an X-ray imaging-based computer assisted surgical device, the system comprising an extended reality device configured to track its 3D pose in a coordinate system, to provide tracking information and to visualize information in the field of view of a user, and a processing unit connected to the extended reality device and configured to execute a computer program product according to claim 12.

14. The system of claim 13, further comprising an input device.

## Patentansprüche

1. Verfahren zum Extended-Reality-Training oder Testen einer röntgenbildbasierten, computerunterstützten Chirurgie-Vorrichtung, wobei das Verfahren die folgenden Schritte aufweist:
Bestimmen einer 3D-Position einer Röntgenbildgebungsvorrichtung in einem Koordinatensystem,
Bestimmen einer 3D-Position eines Objekts in dem Koordinatensystem und
Erzeugen eines simulierten Röntgenprojektionsbildes des Objekts, wobei das simulierte Röntgenprojektionsbild auf der Grundlage der ermittelten 3D-Position des Röntgenbildgebungsgeräts und auf der Grundlage der ermittelten 3D-Position des Objekts erzeugt wird,
Interpretieren des simulierten Röntgenprojektionsbildes und
Bereitstellen von Informationen für einen Benutzer durch eine Extended-Reality-Vorrichtung, wobei die Extended-Reality-Vorrichtung so konfiguriert ist, dass sie ihre 3D-Position im Koordinatensystem verfolgt, wobei die bereitgestellten Informationen auf der Interpretation des simulierten Röntgenprojektionsbildes basieren.

2. Verfahren nach Anspruch 1, wobei der Schritt des Interpretierens des simulierten Röntgenprojektionsbildes mindestens einen Schritt aus der Gruppe umfasst, aufweisend (i) Bestimmen eines geometrischen Aspekts des Objekts, (ii) Anwenden eines Modells des Objekts, (iii) Identifizieren eines Punkts von Interesse, (v) Messen von Dimensionen, (vi) Messen von Abständen, (vii) Berücksichtigen von Objekteigenschaften und (viii) Berücksichtigen von Bildgebungseigenschaften.

3. Verfahren nach einem der Ansprüche 1 und 2, wobei der Schritt des Interpretierens des simulierten Röntgenprojektionsbildes das Bestimmen einer räumlichen Beziehung zwischen mindestens einem abgebildeten Objekt und/oder der Röntgenbildgebungsvorrichtung aufweist.

4. Verfahren nach Anspruch 3, das ferner den Schritt des Vergleichens der räumlichen Beziehung zwischen dem mindestens einen abgebildeten Objekt und/oder der Röntgenbildgebungsvorrichtung mit der ermittelten 3D-Position der Röntgenbildgebungsvorrichtung und/oder mit der ermittelten 3D-Position des Objekts aufweist.

5. Das Verfahren nach einem der Ansprüche 1 bis 4, das ferner den Schritt des Justieren der Bestimmung der 3D-Position der Röntgenbildgebungsvorrichtung und/oder der Bestimmung der 3D-Position des Objekts auf der Grundlage der Interpretation des simulierten Röntgenprojektionsbildes aufweist, wobei die dem Benutzer bereitgestellten Informationen auf der Grundlage der Interpretation des simulierten Röntgenbildes angepasst werden.

6. Das Verfahren nach einem der Ansprüche 1 bis 5, das ferner die Schritte
des Bestimmens einer Darstellung der Röntgenbildgebungsvorrichtung und/oder des Objekts aufweist, wobei die Darstellung auf der verfolgten 3D-Position der Extended-Reality-Vorrichtung und auf der bestimmten 3D-Position der Röntgenbildgebungsvorrichtung und/oder auf der bestimmten 3D-Position des Objekts basiert, und
des Visualisierens der Darstellung durch die Extended-Reality-Vorrichtung.

7. Die Verfahren nach einem der Ansprüche 1 bis 6, wobei das Objekt mindestens ein Objekt aus der Gruppe ist, bestehend aus (i) mindestens einem Teil einer virtuellen Anatomie, (ii) mindestens einem Teil eines realen Anatomiemodells, (iii) mindestens einem Teil eines virtuellen Werkzeugs, (iv) mindestens einem Teil eines realen Werkzeugs, (v) mindestens einem Teil eines realen Implantats, (vi) mindestens einem Teil eines virtuellen Implantats, und wobei das Röntgenbildgebungsgerät ein reales Röntgenbildgebungsgerät oder ein virtuelles Röntgenbildgebungsgerät ist.

8. Das Verfahren nach Anspruch 7, das ferner den Schritt des Verfolgens des mindestens einen Objekts und/oder der Röntgenbildgebungsvorrichtung durch die Extended-Reality-Vorrichtung aufweist, wenn das mindestens eine Objekt ein reales Objekt und/oder die Röntgenbildgebungsvorrichtung eine reale Röntgenbildgebungsvorrichtung ist.

9. Das Verfahren nach einem der Ansprüche 1 bis 8, wobei die dem Benutzer bereitgestellten Informationen mindestens eine Information aus der Gruppe umfasst, bestehend aus (i) einem Hinweis, wie das Röntgenbildgebungsgerät neu positioniert werden soll, (ii) einer Visualisierung einer Position einer virtuellen Bildebene des Röntgenbildes, (iii) eine Visualisierung einer Position einer virtuellen Bildebene eines zuvor simulierten Röntgenbildes, (iv) einen Hinweis für einen nächsten Schritt des Verfahrens.

10. Verfahren nach einem der Ansprüche 1 bis 9, das ferner die Schritte des Empfangens einer Eingabe und des Aktualisierens der dem Benutzer bereitgestellten Informationen auf der Grundlage der Eingabe aufweist.

11. Verfahren nach einem der Ansprüche 1 bis 10, wobei die Darstellung der Röntgenbildgebungsvorrichtung und/oder des Objekts durch die Extended-Reality-Vorrichtung visualisiert wird, so dass die Darstellung als Überlagerung auf der Röntgenbildgebungsvorrichtung und/oder dem Objekt sichtbar ist, wenn das Objekt ein reales Objekt und/oder die Röntgenbildgebungsvorrichtung eine reale Röntgenbildgebungsvorrichtung ist, die durch die Extended-Reality-Vorrichtung sichtbar ist.

12. Ein Computerprogrammprodukt, das Sätze von Anweisungen enthält, die eine Verarbeitungseinheit veranlassen, die Schritte des Verfahrens gemäß einem der Ansprüche 1 bis 11 auszuführen, wenn das Computerprogrammprodukt auf der Verarbeitungseinheit ausgeführt wird.

13. System für das Extended-Reality-Training mit oder das Testen einer röntgenbildbasierten, computergestützten Chirurgie-Vorrichtung, wobei das System eine Extended-Reality-Vorrichtung aufweist, die so konfiguriert ist, dass sie ihre 3D-Position in einem Koordinatensystem verfolgt, Verfolgungsinformationen bereitstellt und Informationen im Sichtfeld eines Benutzers visualisiert, sowie eine Verarbeitungseinheit, die mit der Extended-Reality-Vorrichtung verbunden und so konfiguriert ist, dass sie ein Computerprogrammprodukt gemäß Anspruch 12 ausführt.

14. Das System nach Anspruch 13, das ferner eine Eingabevorrichtung aufweist.

## Revendications

1. En procédé de formation ou de test en réalité étendue d'un dispositif chirurgical assisté par ordinateur basé sur l'imagerie radiographique, le procédé comprenant les étapes suivantes :
déterminer une pose 3D d'un dispositif d'imagerie radiographique dans un système de coordonnées,
déterminer une pose 3D d'un objet dans le système de coordonnées, et
générer une image de projection radiographique simulée de l'objet, dans lequel l'image de projection radiographique simulée est générée sur la base de la position 3D déterminée du dispositif d'imagerie radiographique et sur la base de la position 3D déterminée de l'objet,
interpréter l'image de projection radiographique simulée, et
fournir des informations à un utilisateur par un dispositif de réalité étendue, dans lequel le dispositif de réalité étendue est configuré pour suivre sa position 3D dans le système de coordonnées, dans lequel les informations fournies sont basées sur l'interprétation de l'image de projection radiographique simulée.

2. Le procédé selon la revendication 1, dans lequel l'étape d'interprétation de l'image de projection radiographique simulée comprend au moins une opération parmi celles consistant à (i) déterminer un aspect géométrique de l'objet, (ii) appliquer un modèle de l'objet, (iii) identifier un point d'intérêt, (v) mesurer des dimensions, (vi) mesurer des distances, (vii) prendre en compte les caractéristiques de l'objet, (viii) prendre en compte les caractéristiques d'imagerie.

3. Le procédé selon l'une quelconque des revendications 1 et 2, dans lequel l'étape d'interprétation de l'image de projection radiographique simulée comprend la détermination d'une relation spatiale entre au moins un objet imagé et/ou le dispositif d'imagerie radiographique.

4. Le procédé selon la revendication 3, comprenant en outre l'étape consistant à comparer la relation spatiale entre au moins un objet imagé et/ou le dispositif d'imagerie radiographique avec la pose 3D déterminée du dispositif d'imagerie radiographique et/ou avec la pose 3D déterminée de l'objet.

5. Le procédé selon l'une quelconque des revendications 1 à 4, comprenant en outre l'étape consistant à ajuster la détermination de la pose 3D du dispositif d'imagerie radiographique et/ou la détermination de la pose 3D de l'objet sur la base de l'interprétation de l'image de projection radiographique simulée, et dans lequel les informations fournies à l'utilisateur sont ajustées sur la base de l'interprétation de l'image radiographique simulée.

6. Le procédé selon l'une quelconque des revendications 1 à 5, comprenant en outre l'étape consistant à déterminer une représentation du dispositif d'imagerie radiographique et/ou de l'objet, dans lequel la représentation est basée sur la pose 3D suivie du dispositif de réalité étendue et sur la pose 3D déterminée du dispositif d'imagerie radiographique et/ou sur la pose 3D déterminée de l'objet, et à visualiser la représentation par le dispositif de réalité étendue.

7. Le procédé selon l'une quelconque des revendications 1 à 6, dans lesquels l'objet est au moins un objet parmi le groupe comprenant (i) au moins une partie d'une anatomie virtuelle, (ii) au moins une partie d'un modèle anatomique réel, (iii) au moins une partie d'un outil virtuel, (iv) au moins une partie d'un outil réel, (v) au moins une partie d'un implant réel, (vi) au moins une partie d'un implant virtuel, et dans lequel le dispositif d'imagerie radiographique est un dispositif d'imagerie radiographique réel ou un dispositif d'imagerie radiographique virtuel.

8. Le procédé selon la revendication 7, comprenant en outre l'étape consistant à suivre au moins un objet et/ou le dispositif d'imagerie radiographique à l'aide du dispositif de réalité étendue, lorsque ledit au moins un objet est un objet réel et/ou que le dispositif d'imagerie radiographique est un dispositif d'imagerie radiographique réel.

9. Le procédé selon l'une quelconque des revendications 1 à 8, dans lequel les informations fournies à l'utilisateur comprennent au moins une information parmi le groupe constitué (i) un conseil sur la manière de repositionner le dispositif d'imagerie radiographique, (ii) une visualisation d'une position d'un plan image virtuel de l'image radiographique, (iii) une visualisation d'une position d'un plan image virtuel d'une image radiographique simulée précédente, (iv) un conseil pour une étape suivante de la procédure.

10. Le procédé selon l'une quelconque des revendications 1 à 9, comprenant en outre les étapes consistant à recevoir une entrée et à mettre à jour les informations fournies à l'utilisateur sur la base de l'entrée.

11. Le procédé selon l'une quelconque des revendications 1 à 10, dans lequel la représentation du dispositif d'imagerie radiographique et/ou de l'objet est visualisée par le dispositif de réalité étendue, de sorte que la représentation est visible sous forme de superposition sur le dispositif d'imagerie radiographique et/ou l'objet, lorsque l'objet est un objet réel et/ou le dispositif d'imagerie radiographique est un dispositif d'imagerie radiographique réel visible à travers le dispositif de réalité étendue.

12. En produit logiciel comprenant des ensembles d'instructions amenant une unité de traitement à exécuter les étapes du procédé selon l'une quelconque des revendications 1 à 11, lorsque le produit logiciel est exécuté sur l'unité de traitement.

13. En système de formation ou de test en réalité étendue d'un dispositif chirurgical assisté par ordinateur basé sur l'imagerie radiographique, le système comprenant un dispositif de réalité étendue configuré pour suivre sa position 3D dans un système de coordonnées, pour fournir des informations de suivi et pour visualiser des informations dans le champ de vision d'un utilisateur, et une unité de traitement connectée au dispositif de réalité étendue et configurée pour exécuter un produit logiciel conformément à la revendication 12.

14. Le système selon la revendication 13, comprenant en outre un dispositif d'entrée.
